# EUROPEAN PATENT APPLICATION

(11) **EP 4 285 820 A1**
(43) Date of publication of application: **06.12.2023**
(21) Application number: 22176104.2
(22) Date of filing: 30.05.2022
(51) Int. Cl.: A61B 5/16, A61M 21/00, A61F 9/04, G04B 23/02, G04C 19/02, A61B 5/00, A61M 21/02

(54) **PATCH FOR SLEEP MONITORING**

(71) Applicant: BIC Violex Single Member S.A., 145 69 Anoixi (GR)
(72) Inventor: Chrysanthakopoulos, Nikolaos, 14569 Anoixi (GR)
(74) Representative: Peterreins Schley

(57) **Abstract**

A patch for eyelid monitoring, comprising a flexible substrate configured for attachment to at least a portion of an external surface of an eyelid, an adhesive layer comprised on a first portion of the flexible substrate, wherein the adhesive layer is configured, in use, to adhere to the external surface of the eyelid, to enable a semi-permanent attachment of the flexible substrate to the external surface of the eyelid, and integrated electronics comprised at least partially within the flexible substrate, and/or comprised on a second portion of the flexible substrate on an opposite side of the flexible substrate to the first portion. The integrated electronics comprises: a sensor configured to generate one or more signals characterising a shape of the flexible substrate, a communications interface configured to communicate the one or more signals to an external apparatus, a power source configured to provide electrical energy to the sensor and the communications interface.

## Description

### Technical Field

The embodiments described herein concern a patch for eyelid monitoring, and an associated computer-implemented method, system, computer program element, and computer readable medium.

### Background

Sleep disorders affect the ability of an individual to sleep well on a regular basis. Dependent on the type of sleep disorder, sufferers may experience difficulty when falling asleep, and may feel tired throughout the following day. Lack of sleep has a negative impact on energy, mood, concentration, and overall health.

Light exposure can hinder transitions between sleep cycles, thus reducing sleep quality. Light plays an important role in regulating the circadian rhythm of an individual. Furthermore, an individual experiences several different phases of sleep during a typical sleep session. Most significantly, Rapid Eye Movement Sleep (REMS) is a unique phase of sleep, characterised by random rapid movement of the eyes, accompanied by low muscle tone throughout the body of the sleeping individual. During REMS, the sleeping individual often experiences vivid dreams.

REMS is physiologically different from other phases of sleep (generically termed NREMS). REMS and NREMS alternate within one sleep cycle, which lasts about 90 minutes in adult humans. As sleep cycles continue, the proportion of REMS experienced by the individual increases. Interference from light exposure can affect the amount and/or quality of REMS. Furthermore, if an individual wakes during REMS, they may feel more tired as opposed to when the individual wakes during NREMS.

There are various approaches to improving sleep quality. For example, a bedroom can be furnished with curtains that are opaquer. Bedroom walls and doors can be made thicker, and double-or triple-glazed windows can be provided. Careful attention to the positioning of light emitting devices within the bedroom can improve sleep quality, and in an extreme case, the individual can wear an eye mask to ensure that access light does not reach their eyelids. Of course, sleeping medication is also an option. Products such as smartwatches and headbands enable the measurement of REMS indirectly, by monitoring body motions or brain activity. However, users may find the use of such solutions to improve sleep quality to be intrusive or undesirable. Accordingly, equipment for improving sleep quality can be further improved.

### Summary

According to a first aspect, a patch for eyelid monitoring, comprises a flexible substrate configured to attached to at least a portion of an external surface of an eyelid, an adhesive layer comprised on a first portion of the flexible substrate, wherein the adhesive layer is configured, in use, to adhere to the external surface of the eyelid, to enable a semi-permanent attachment of the flexible substrate to the external surface of the eyelid. The patch further comprises integrated electronics comprised at least partially within the flexible substrate, and/or comprised on a second portion of the flexible substrate on an opposite side of the flexible substrate to the first portion. The integrated electronics comprises a sensor configured to generate one or more signals characterising a shape of the flexible substrate, a communications interface configured to communicate the one or more signals to an external apparatus, and a power source configured to provide electrical energy to the sensor and the communications interface.

An effect of the patch according to the first aspect is that the patch provides direct, comfortable, and precise monitoring of the sleep phase of a user, for example by monitoring REM eye movements referred to the surface of the eyelid of an individual.

Accurate monitoring of the sleep phase of a user enables the wake-up time of a user to be adjusted to avoid waking user up during a REM phase.

Furthermore, in embodiments, the patch directly monitors external lighting conditions at the eyelids of the user. Accordingly, a more accurate estimate of light intensity at the user's eyelids as possible, as opposed to monitoring the ambient light intensity via a proxy measure such as time of day, or a light meter placed elsewhere in a room that the individual is sleeping in. In further embodiments, sleep quality may be improved because the patch can be used to block ambient light during REM phases by means of a configurable electrochromic layer, for example.

According to second aspect, a computer-implemented method for sleep monitoring comprises:
- obtaining one or more signals characterising a shape of an eyelid from a patch according to the first aspect, or embodiments of the first aspect described herein;
- analyzing the one or more signals to determine whether the one or more signals characterize a first or a second phase of sleep of a user; and
- performing at least one output action based on the analysis resulting in a determination that a user of the patch is experiencing either of the first or second phases of sleep.

According to a third aspect a system comprises at least one patch according to the first aspect, or embodiments of the first aspect described herein; and an apparatus. The apparatus comprises a processor, a communications interface, and a memory.

The communications interface of the apparatus is configured to obtain one or more signals characterising a shape of an eyelid from the at least one patch. The processor is configured to analyze the one or more signals to determine whether the one or more signals characterize a first or a second phase of sleep of a user. The processor is configured to transmit a command to perform at least one output action based on the analysis resulting in a determination that a user of the patch is experiencing either of the first or second phases of sleep.

According to a fourth aspect a computer program element comprises machine readable instructions which, when executed by a processor, causes the processor to perform the computer implemented method according to the first aspect, or embodiments of the third aspect described herein.

According to a fifth aspect a computer readable medium comprises the computer program element according to the fourth aspect.

### BRIEF DESCRIPTION OF THE DRAWINGS

Other characteristics will be apparent from the accompanying drawings, which form a part of this disclosure. The drawings are intended to further explain the present disclosure and to enable a person skilled in the art to practice it. However, the drawings are intended as nonlimiting examples. Common reference numerals on different figures indicate like or similar features.
- **Figure 1a**: schematically illustrates a plan view of an exemplary patch.
- **Figure 1b**: schematically illustrates a section view of the exemplary patch of **Figure 1a**.
- **Figure 2**: schematically illustrates a plan view an exemplary patch comprising an extension portion.
- **Figures 3a-e**: illustrates examples of patch shapes.
- **Figure 3f**: illustrates an example packaging strip comprising a plurality of patches
- **Figures 4a-c**: illustrate an example of detection of a REM pattern using a patch.
- **Figures 5a-b**: illustrates an example of histograms for detecting a REM pattern.
- **Figure 6**: schematically illustrates a section view of an example further patch design comprising additional layers.
- **Figure 7**: illustrates a concept according to an example.
- **Figure 8**: illustrates an example of an apparatus.
- **Figure 9**: illustrates an example of a method according to an example of the second aspect.
- **Figure 10**: illustrates an example of a system according to an example of the third aspect.
- **Figure 11**: illustrates an example of the system according to the third aspect, in use.
- **Figures 12a-b**: illustrate an example of a graphical user interface.
- **Figure 13**: illustrates an example of a hypnogram.

### DETAILED DESCRIPTION

When an individual closes their eyes to sleep, each eyelid comes into close contact with a corresponding eyeball (or the ocular surface of the eyeball). Specifically, the ocular surface makes contact with the upper and lower eyelids. The ocular surface is not perfectly spherical, but deviates from the form of a perfect sphere in the region of the cornea. Therefore, when the eyelids of a sleeping individual are closed, the surface of each eyelid is stretched and deformed based on the position of the eyeball.

In a study performed on young adults, the mean pressure exerted by an eyeball of a on the eyelid has been measured as an 8.0 ± 3.4 mm Hg ("Eyelid Pressure and Contact with the Ocular Surface" by A. Shaw, M. Collins, B. Davis, and L. Carney in Investigative Ophthalmology & Visual Science April 2010, Vol.51, 1911-1917, doi:https://doi.org/10.1167/iovs.09-4090).

REM sleep is important for memory consolidation, and is the key to experiencing a refreshing night of sleep. During REM sleep, the brain actively converts waking experiences into lasting memories. REM helps growing brains adjust the strength or number of neuronal connections to match the input they receive from their environment.

The present specification proposes to monitor the deformation of at least one eyelid of an individual using an adhesive patch attached to the eyelid, in order to identify a point at which a REM sleep phase of an individual has started, or ended, for example. There is a growing trend towards embedding microsensors and electronic components in flexible E-skin polymers for wearable, or on-skin devices. For example, stretchable self-powered sensors, flexible organic photodetector films, flexible electrochromic devices, stretchable audio transducers, stretchable light emitting devices, and near field communication (NFC) can be integrated to provide discreet sleep monitoring sensors.

Although, in the following description, one eyelid patch is described, a skilled person will appreciate that in some deployments, the use of two eyelid patches may be necessary. For example, REM monitoring of the eyeball can be carried out with one eyelid patch 20. However, applications involving controlling the amount of light reaching the eyes of a user may use two eyelid monitoring patches 20. Furthermore, although the present specification assumes that the described patch or patches 20 can be applied to the upper eyelid of a user, a skilled person will understand that the patches may also be applied to a lower eyelid of user for detection, for example, of the REM sleep state.

According to a first aspect, a patch 20 for eyelid monitoring comprises a flexible substrate 22 configured for attachment to at least a portion of an external surface 10 of an eyelid 12, and an adhesive layer 24 comprised on a first portion 22a of the flexible substrate 22. The adhesive layer 24 is configured, in use, to adhere to the external surface 12 of the eyelid 10, to enable a semi-permanent attachment of the flexible substrate 22 to the external surface of the eyelid. The patch 20 further comprises integrated electronics 26 comprised at least partially within the flexible substrate 22, and/or comprised on a second portion 22b of the flexible substrate on an opposite side of the flexible substrate to the first portion.

The integrated electronics 26 comprises a sensor 28 configured to generate one or more signals characterising a shape of the flexible substrate 22, a communications interface 30 configured to communicate the one or more signals to an external apparatus 50, and a power source 32 configured to provide electrical energy to the sensor 28 and the communications interface 30.

In an example, the signals are analogue signals output by the sensor 28. In an example, the signals are data (when the sensor 28 is configured to output its measured parameter in digital form).

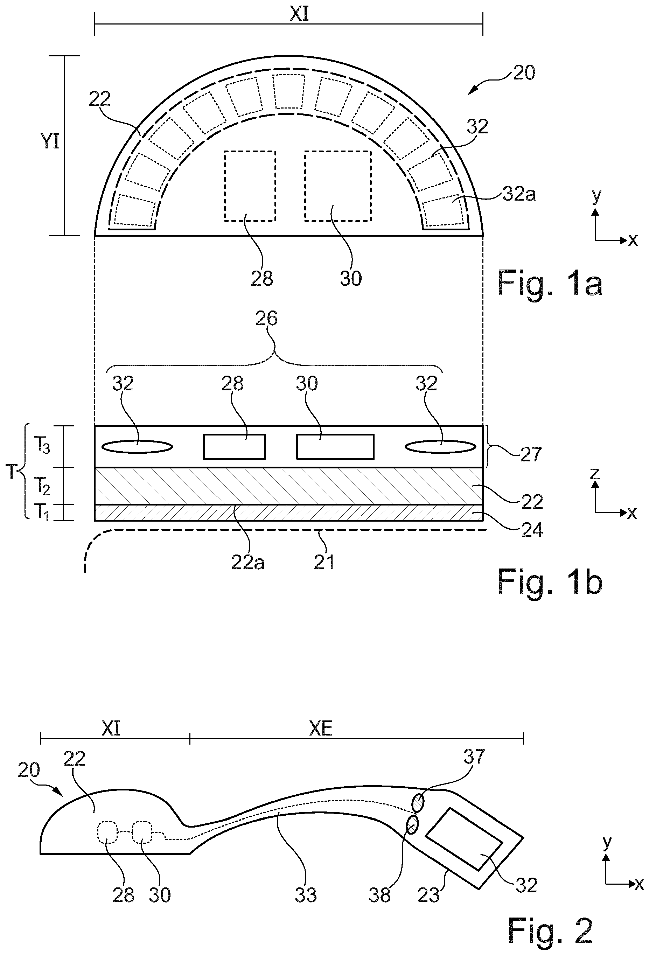

**Figure 1a** schematically illustrates a plan view of a patch 20.

**Figure 1b** schematically illustrates a section view of the patch 20 of **Figure 1a**.

In embodiments, the eyelid patch 20 may variously be suitable for adhering softly to the external side of the user's eyelid, and be capable of detecting sleep patterns of the user, changing its opacity, illuminating, and communicating with mobile devices, for example.

The flexible substrate 22 of the patch is intended to be adhesively attached to an eyelid of user using the adhesive layer 24. The flexible substrate 22 is capable of conforming to the shape of the eyelid, in use. Eyelid patches or eyelid lifting patches for cosmetic use comprise breathable, waterproof, and transparent materials. In an example, the flexible substrate 22 comprises a transparent medical grade material. For example, the flexible substrate 22 comprises a polymeric film, such as polyurethane, polyester, polyvinyl acetate, silicone, silicone elastomers such as polydimethylsiloxane and the like.

The flexible substrate should be able to transfer physical deformations of an eyelid caused by the movement of the corneal region of the eye during, for example, REMS.

The illustrated flexible substrate 22 has a flattened semi-circular shape (hemi-spherical), in order to provide a good fit for most human eyelid shapes. However, there exists a wide variation in human eyelid shapes, and the flexible substrate 22 can be provided in many other shapes, such as hemi-elliptical, tori-spherical, or rectangular.

A first side 22a of the flexible substrate 22 (the reverse side of the flexible substrate 22, in other words, the side of the flexible substrate 22 intended to be closest to the user's eye, when fitted to the user's eyelid) comprises an adhesive layer 24. The adhesive layer 24 is applied to the first side 22a of the flexible substrate 22 in sufficient quantities to ensure adhesion of the flexible substrate 22 to the eyelid. Furthermore, the adhesive comprised in the adhesive layer 24 is a semi-permanent adhesive facilitating easy removal of the patch 20 from the user's eyelid after a period between 1 and 24 hours, without injuring the user's eyelid. In an example, the adhesive comprised in the adhesive layer 24 is water-soluble, to facilitate removal of the patch 20. For example, the adhesive comprised in the adhesive layer 24 may be a hydrocolloid such as gelatin or collagen. The adhesive may be a synthetic rubber, silicon, or other medical adhesive.

In an embodiment, the adhesive layer is integral to the flexible substrate 22. For example, the first portion 22a of the flexible substrate that is configured to contact the surface of the eyelid can be treated, for example by processing the first portion 22a of the flexible substrate 22 using a plasma treatment, to enhance the ability of the first portion 22a of the flexible substrate to adhere to skin. In this case, the adhesive layer 24 is integral to the flexible substrate 22.

A stretchable nano membrane for skin electronics is discussed in *"*Highly conductive and elastic nanomembrane for skin electronics" by Dongjun Jung, et al, in "Science", 2021; 373 (6558): 1022 DOI: 10.1126/science.abh4357. The flexible substrate 22 may comprise such a stretchable nano membrane.

For example, the stretchable conductor features ultra-thin thickness, metal-like conductivity, high stretchability, and an ease of patternability. The composite material comprises metal nano wires that are tightly packed in a monolayer within ultra-thin rubber film. This material was made using a process based on float assembly.

In an example, the adhesive layer 24 is applied to substantially the entire first side 22a of the flexible substrate to provide maximum grip to the user's eyelid. In an example, the adhesive layer 24 is applied to portions of the first side 22a, for example in a dotted or striped pattern.

In an example, the patch 20 comprises a backing sheet 21, intended to protect the adhesive layer 24 from the effects of oxidation or physical damage before the time of use. A similar purpose may be served by a carrier medium, from which the patch 20 is peeled before application to a user.

In an example, the eyelid patch is removable, and/or reusable. In an embodiment, the eyelid patch is waterproof and/or washable.

In Figures 1a and 1b, a dimension X1 defines the greatest width of the patch 20, or in other words, the greatest dimension of the patch 20 in a direction substantially corresponding to, or substantially parallel with, the edge of an upper eyelid of a user, in use. A dimension Y1 defines the greatest length of the patch 20, or in other words, the greatest dimension of the patch 20 in a direction substantially perpendicular to the edge of an upper eyelid of a user, in use. A dimension T corresponds to the thickness of the patch 20, or in other words, how far the patch 20 protrudes from the surface of the upper eyelid of a user, in use.

In an example, the dimension XI is in the range 1 - 5 centimetres. In an example, the dimension YI is in the range 1 - 5 centimetres. A skilled person will appreciate that the eventual dimensions XI and YI can be adapted based on the eventual form factor of the patch 20, and the age range of intended users.

The eyelid patch 20 of **Figure 1b** further comprises integrated electronics 26 comprised in an encapsulation 27. In this example, the encapsulation 27 is, for example, a stretchable and transparent silicone rubber surround, ensuring that the integrated electronics 26 are protected from accidental damage during application or unpackaging of the patch 20.

According to another embodiment, the encapsulation 27 may be omitted, and the integrated electronics 26 are partially, or wholly embedded in the flexible substrate 22. Although the encapsulation 27 is illustrated as a layer having constant thickness T₃, the encapsulation 27 can have an arbitrarily-shaped cross section (in the x-z plane, and/or the x-y plane). For example, the encapsulation 27 can be in the form of a teardrop or ogivoid. This may make the patch 22 easier to apply or remove, and may accommodate larger items of the integrated electronics 26.

The total thickness of the eyelid patch 20 T is the sum of the thickness of the adhesive layer T₁, the thickness of the flexible substrate 22 T₂, and the thickness of the encapsulation 27 T₃ (if present). In examples, the total thickness T of the eyelid patch is in the range 0.3mm to 4mm.

According to an embodiment, each of the flexible substrate 22, the adhesive layer 24, and the encapsulation 27 (if present) are translucent or transparent to incident light impinging on the patch 20 from the direction of the second side 22b of the flexible substrate.

According to an embodiment, at least one of the flexible substrate 22, the adhesive layer 24, and the encapsulation 27 (if present) comprise a die or stain, so that the baseline colour of the eyepatch can be customised to match a range of appropriate skin tones. According to an embodiment, the baseline colour visible at the second side 22b of the patch 20 can be selected from any colour of the visible spectrum. According to an embodiment, the baseline colour may be homogeneous, or can be patterned. For example, a patch intended for use with the left eyelid may comprise a "L" pattern or letter, and a patch intended for use with the right eyelid may comprise a "R" pattern or letter.

The patch 20 may comprise more than the illustrated number of layers. For example, the flexible substrate 22 may comprise two, three, four, five or more layers of the same or different flexible substrate material. Each layer may have the same or different properties, functions, electrical components from another layer, in a subsequent or different position of the eyelid patch.

The flexible substrate 22 (stretchable supporting polymer layer) and encapsulation layer 27 (if present) can be manufactured using one or more of: chemical synthesis, electro spinning, coating, sputtering, printing, transfer patterning, liquid-phase blending, filtration, laser micro-machining, carbonisation, dip-coating, bar coating, ultrasonication, and/or vacuum filtration.

As previously noted, the integrated electronics 26 can be attached to, or partially embedded in, the second surface 22b of the flexible substrate 22. In an embodiment, an encapsulation layer 27 is provided that surrounds the components of the integrated electronics 26.

The integrated electronics 26 comprise a sensor 28 configured to generate one or more signals characterising a shape, or a change of shape, of the flexible substrate 22. Therefore, the sensor 28 is an eyelid shape sensor, because the flexible substrate 22 refers movement of an attached eyelid to the sensor 28. The sensor 28 can, therefore, be considered to be usable to detect REM by detecting one or more of strain, deformation, or pressure of the flexible substrate 22.

According to an embodiment, the sensor 28 is a single sensor, a 1D array of sensors, or a 2D array of sensors. According to an embodiment, the sensor is a strain sensor, or a 2D strain sensor. According to an embodiment, the sensor 28 comprises one, or a 1D or 2D array of piezoelectric sensors, such as piezoelectric nanoribbons, integrated on the flexible substrate 22. According to an embodiment, sensor 28 comprises one, or a 1D or 2D array, of MEMs strain gauges. According to an embodiment, the sensor 28 comprises one, or a 1D or 2D array of carbon nanotube mesh sensors or carbon nanotube film sensors.

During REMS, eyeballs move rapidly with, for example, 16 motions per minute on average. According to scientific research, the average eyeball rotation angle is 7°, and the average speed of rotation is 59° per second. The closed eyelid is typically in contact with the eyeball at a contact width ranging from 0.44mm to 0.76mm. The associated deformation of the eyelid may be detected by at least the sensor 28 of the integrated electronics 26. In an embodiment, the sensor 28 is at least one resistive sensor, at least one capacitive sensor, at least one piezoelectric sensor, at least one triboelectric sensor, or at least one graphene-based strain sensor (using crumpled graphene, for example).

In examples, the strain sensor is fabricated from carbon nano materials, carbon nanotubes, graphene and graphene derivatives, metal nano wires, nano fibres, nanoparticles, MXenes (for example, two-dimensional inorganic compounds such as Ti₃C₂Tₓ), ionic liquids, hybrid micro and nano structures, conductive polymers, thin films, or conductive yarns and fabrics.

In an embodiment, a region of the patch 20 comprising the sensor 28 can be incorporated into the flexible substrate 22.

Of course, the stretchability of the sensor 28 depends on the type of matrix or substrate, the fabrication process, and the aspect ratio of micro or nano materials used as sensing elements. In embodiments, the stretchability of the flexible strain sensors varies from 2% to 1400%. In embodiments, the gauge factor of the sensitivity of the strain sensor depends on sensing elements, microstructure, the sensing mechanism, and the fabrication process may vary from 1.25 to 80,000. The article *"*Wearable and Stretchable Strain Sensors: Materials, Sensing Mechanisms, and Applications" by Hamid Souri et. al in "Advanced Intelligent Systems", 11 June 2020, 2, 2000039, https://doi.org/10.1002/aisy.202000039 discusses a range of wearable resistive, capacitive, and optical strain sensors suitable for integration onto the flexible substrate 22 of the present specification and associated gauge factors.

In an embodiment, the response time of the sensor 28 is defined as the amount of time needed for the sensor 28 to reach a steady state response following a perturbation caused by the movement of the cornea underlying an eyelid that is being sensed. In examples, the response time varies between 0.2 ms to 320 ms.

The dynamic durability is the endurance of the sensor 28 to long term stretching and releasing. The sensor 28 should have a dynamic durability of at least 1000 cycles, and preferably at least 20,000 cycles.

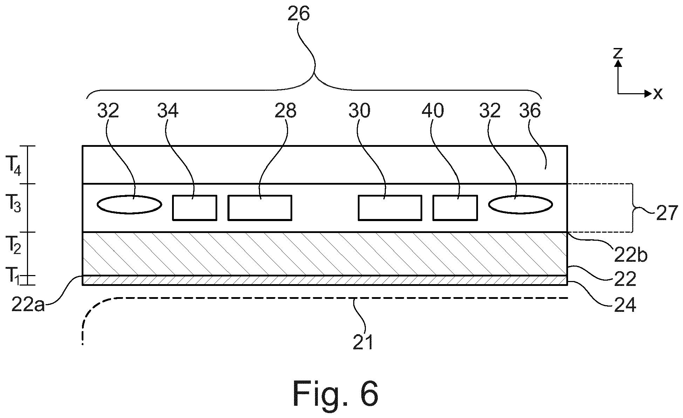

An example of the characterisation of sleep states, for example REMS, as discussed in connection with **Figure 6** below

The power source 32 of the patch 20 is configured to supply enough electrical power to the other components comprised in the integrated electronics of the patch to enable normal, or standby functionality as discussed in embodiments of this specification. In an embodiment, the power source 32 is configured to generate power from eye movements or from incident light, and to store the generated power on the patch 20.

In embodiments, the power source 32 of the patch 20 is configured to receive electrical energy from an external source using a wired or wireless transmission modality. For example, when an extension portion 23 of the patch 20 is provided, a thin power wire 33 is electrically coupled to the components of the integrated electronics 26 on the eyelid-contacting section XI of the patch 20. A power source 32 can be provided on the distal section of extension portion 23. Therefore, power source 32 of the patch 20 is, in examples, an energy generation and storage component, or a buffer configured to receive energy from an external wired or wireless power source. For example, a wired power source may be a disposable battery such as lithium battery, another type of rechargeable battery, or a super capacitor. The rechargeable battery or super capacitor may be charged via pogo pins, a wireless RF charging coil, or a USB connection.

In embodiments, an extension portion 23 comprises an antenna (not shown) for receiving wireless power, for example using the "Qi" inductive charging standard of the Wireless Power Consortium. In another embodiment, an extension portion 23 may be omitted, and the power source 32 provided on the patch 20 may comprise an antenna capable of receiving wireless power. In an example, the patch 20 is configured to receive wireless power from a wireless power transmitter on a proximate bedside table, or from a wireless power transmitter installed in a bed headrest.

In embodiments, the power source 32 of the patch is a nano-generator configured to self-generate and store sufficient electrical energy to perform sensing and data communication operations from the patch 20. Self-powered sensors capable of harvesting energy from human breathing and motion for use in wearable applications are discussed, for example, in the journal article *"*High-energy all-in-one stretchable micro-supercapacitor arrays based on 3D laser-induced graphene foams decorated with mesoporous ZnP nanosheets for self-powered stretchable systems" Cheng Zhang, et. al, Nano Energy, 2021; 81: 105609 DOI: 10.1016/j.nanoen.2020.105609.

In embodiments, the nano-generator comprises a plurality of micro supercapacitor cells arranged on the flexible substrate 22. For example, the micro supercapacitor cells are arranged in a serpentine (island-bridge) pattern allowing the configuration to stretch and bend, while reducing deformation of the islands comprising the micro supercapacitors. When combined, such a structure provides a micro supercapacitor array. This increases stretchability of the patch 20, and enables flexible choice of voltage output based on the interconnection topology of the micro supercapacitor array.

In embodiments, each micro supercapacitor cell comprised in the nano-generator has a hybrid electrode fabricated from ultra-thin ZnP nano sheets, provided on a graphene foam. As discussed in the Nano Energy paper cited above, such nano-generators can charge and discharge efficiently using, for example, a triboelectric generator, a piezoelectric generator, or a thermoelectric generator integrated into the power source 32 region of the patch 20, with energy that is generated stored until needed to power the integrated electronics of the patch 20.

Referring to **Figure 1****,** one example of a distributed micro supercapacitor power source 32 having the shape of a crescent around the outer edge of a hemi-elliptical flexible substrate 22 is illustrated. A plurality of micro supercapacitor generator cells 32a are shown in dotted form inside the power source 32 region.

In an embodiment, the nano-generator comprises a triboelectric generator (TENG).

In an embodiment, the nano-generator comprises a piezoelectric generator (PENG).

In an embodiment, the nano-generator comprises a thermoelectric generator (TEG).

For example, the power source 32 may comprise a micro supercapacitor configured to store the electrical energy generated by the nano-generator.

In embodiments, the nano-generator provides an output voltage ranging from 3 V to 120 V. In embodiments, the nano-generator provides a current density ranging from 5 to 20 *µ*A cm⁻². In embodiments, the nano-generator has a power density ranging from 50 to 500 *µ*W cm⁻².

The integrated electronics 26 of the patch 20 further comprises a communications interface 30. This is a component capable of communicating data at least including information about, or derived from, motion data of the sensor 28. According to an embodiment, the communications interface 30 is a low-energy wireless communication protocol capable of transmitting the motion data of the sensor 28 a short distance to an external apparatus, thus limiting the power required to transmit the motion data.

In embodiments, the communications interface 30 uses Near Field Communications (NFC), RFID, Bluetooth (TM), or Zigbee (TM).

In embodiments, the individual elements of the integrated electronics 26 are electrically connected using micro wire traces deposited (for example, by plasma vapour deposition) on the surface, or within, the flexible substrate, for example. By depositing layers comprising the sensor 28, the communications interface 30, and the power source 32 so that they are connected by the micro wire traces, each component of the integrated electronics 26 are electrically connected.

**Figure 2** schematically illustrates a plan view a patch comprising an extension portion.

According to an embodiment, the flexible substrate 22 further comprises an extension portion 23 that that does not contact the eyelid, in use, and at least one of the power source, and/or the communications interface are comprised on the extension portion 23.

In some cases, an alternative method for integrating specific components onto the eyelid contacting portion of the patch 20 could be provided. The embodiment of **Figure 2** illustrates a patch 20 comprising an extension portion 23. The extension portion 23 can be made of the same material as the flexible substrate 22. Alternatively, the extension portion 23 can be made of a flexible and more durable, or thicker material as compared to the flexible substrate 22 of the patch 20. A patch 20 comprising extension portion 23 therefore provides extra mechanical support to the portion of the patch 20 contacting the eyelid, and can accommodate extra electrical circuitry.

The portion of the patch 20 of **Figure 2** intended to contact the eyelid (indicated by the linear extent of XI) comprises, in this example, integrated electronics 26 comprising an eyelid shape sensor 28 and a communications interface 30. A wire 33 connects the integrated electronics 26 to a power source 32 (such as a battery) positioned on a distal end of the extension portion XE. The distal end of the extension portion XE can also comprise an audio feedback device 37, or a haptic feedback device 38. In an example, a further wire following the path of wire 33 is a radio communications antenna.

The distal end of the extension portion XE can be configured to adhere to a temple, eyebrow, nose, or forehead of a user.

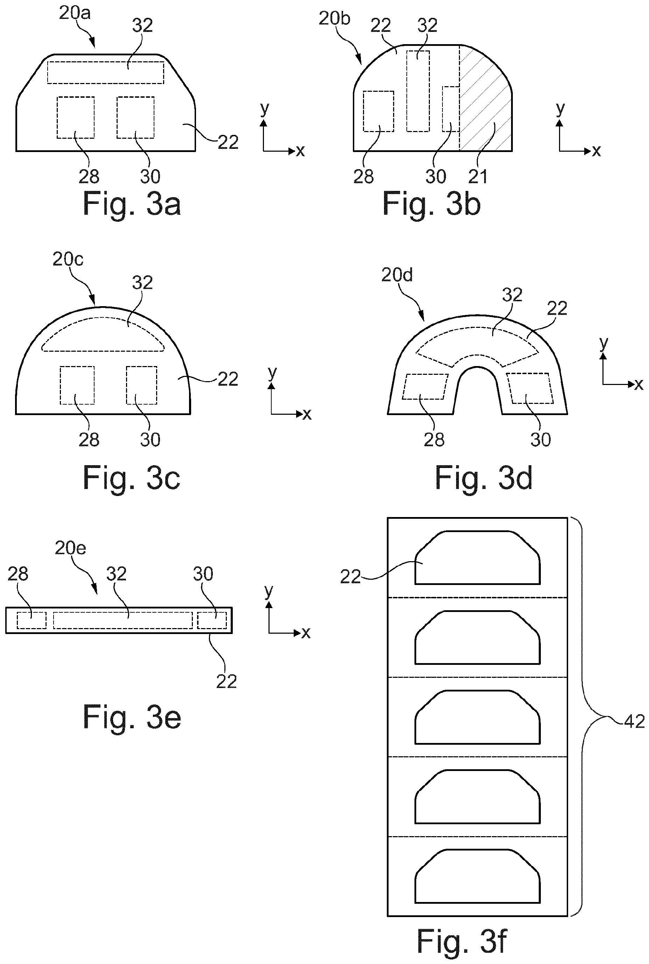

**Figures 3a****-e** illustrates examples of alternative patch shapes.

**Figure 3a** illustrates a patch 20a comprising a rectangular extension accommodating the sensor 28 and the communications interface 30, with the power source 32 accommodated in a curved portion of the 20a. The flexible substrate 22 of this patch covers a large area of a user's eyelid, which may enable more electrical power to be harvested from the user's eyelid, and to enable a better fidelity when detecting REMS.

**Figure 3b** illustrates a patch 20b comprising a rectangular extension and a curved portion. The power source 32 is aligned along the y-axis at the longest dimension of the patch 20b. Each of the sensor 28 and the communications interface 30 are located on either side of the power source 32. In some situations, or for some nano-generator types, positioning the power source 32 differently may enable more efficient energy extraction. The patch 20b is also provided with an optional backing sheet 21 for protection of the adhesive layer 24. The flexible substrate 22 of this patch covers a large area of a user's eyelid, which may enable more electrical power to be harvested from the user's eyelid, and to enable a better fidelity when detecting REMS.

**Figure 3c** illustrates a hemi-elliptical patch 20c, where the power source 32 is concentrated in the tip of the hemi-ellipse. For example, portions of the eyelid closer to the upper or lower eyelid crease may experience more frequent stretching, or stretching of greater magnitude, and thus may be suitable to provide motions to power the power source 32.

**Figure 3d** illustrates a hemi-elliptical patch 20d comprising a central notch. The sensor 28, communications interface 30, and power source 32 are distributed along the extent of the hemi-elliptical patch 20d. The central notch can, for example, reduce tension of the flexible substrate 22 when attempting to fit the patch 20d to a user, and thus the hemi-elliptical patch 20d can fit a wider range of eyelid shapes and sizes.

**Figure 3e** illustrates a thin rectangular patch 20e having an aspect ratio, for example, in the range of'1:2 to 1:20, preferably 1:10. The sensor 28, communications interface 30, and power source 32 are distributed along the length of the rectangular patch 20d. Although the flexible substrate 22 of this patch covers a smaller area of a user's eyelid, the sensor 28 can still detect some movement of an attached eyelid, and thus REMS can still be distinguished.

**Figure 3f** illustrates an example packaging strip 42 comprising a plurality of patches. For reasons of hygiene, or owing to slow degradation of the function of nano-fabricated components of a patch 20 during use, it may be desirable to sell patches 20 on a strip 42, a roll, or another substrate format. This enables the user to peel off a new patch 20 (or pairs of patches 20) before applying the patch at the beginning of a sleep session.

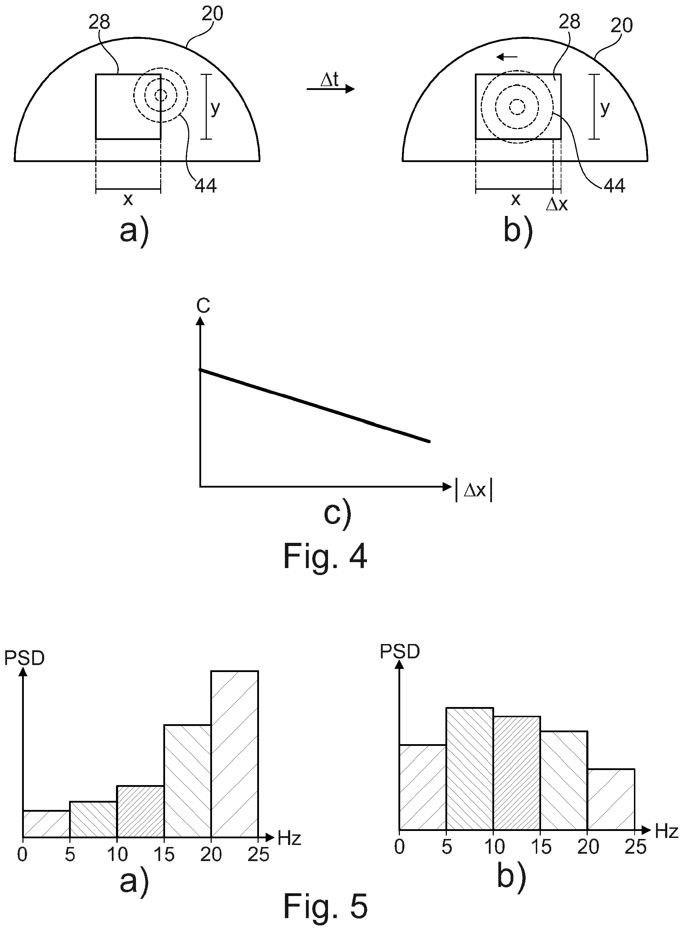

**Figures 4a****-c** illustrates an example of detection of a REM pattern using a patch.

According to an embodiment, the sensor 28 is configured to detect motion of the external surface of the eyelid caused by a motion of an eyeball.

According to an embodiment, the sensor 28 is a strain sensor configured to generate the one or more signals characterising strain induced in the flexible substrate 22 owing to deformation of the eyelid 10 caused by movement of an underlying eyeball 12, in use.

According to an embodiment, the strain of the flexible substrate 22 is characterised as a scalar quantity. For example, the average change in resistance or capacitance of the entire sensor 28 when experiencing a change of applied strain. Analyzing the spectrum of this signal can provide enough information (such as a spectral signature) characterizing the presence of REMS, for example.

According to an embodiment, the strain of the flexible substrate 22 is characterised as a vector quantity. For example, the sensor 28 may comprise a plurality of detection cells (not illustrated). The sensor is configured to provide a separate strain reading for each detection cell. In the case of a sensor 28 comprising a 1D row of detection cells, an X or Y component of eyeball movement can be characterized. In the case of a sensor 28 comprising a 2D matrix of detection cells, the X and Y components of eyeball movement can be characterized. This enables more complicated classifier-based REMS detection approaches to be applied.

**Figure 4a** schematically illustrates a patch 20 and its associated sensor 28 (other components of the patch 20 are present but not shown to aid clarity) at a first point in time during REM, when fixed to an eyelid 10. As one example, the sensor 28 is a 2D array of micro supercapacitor cells, enabling the resolution of eyelid motion into two dimensions. This is useful because during REMS, and eye often traces a circular, or arc like trajectory. However, a 1D array or strip of micro supercapacitor cells can often provide enough resolution of the eye movement to obtain, for example, spectral content of the motion of the underlying deformation of the eyelid, thus characterising the REM. In **Figure 4a****,** the deformation of the patch 20 caused by an underlying corneal protrusion of the eye deforms the sensor 28 at a first x-y coordinate of the patch 20.

**Figure 4b** illustrates the patch 20 and its associated sensor 28 at a second point in time during REM, when fixed to an eyelid 10. Owing to the REM, the underlying eyeball has moved, and the underlying corneal protrusion of the eye deforms the sensor 28 towards a second x-y coordinate of the patch 20. The flexible substrate 22 and corresponding sensor 28 therefore experiences stretching which enlarges the size of the sensor 28 along the x-axis of the sensor 28 by the indicated amount Δx.

**Figure 4c** illustrates an example of the change in electrical characteristic of the sensor 28 owing to the movement defined in **Figures 4a** and **4b****.** If the sensor 28 comprises micro supercapacitor cells, one example of an effect of an enlargement (stretching) of the sensor 28 on its electrical characteristics is a reduction in the overall capacitance of the micro supercapacitor cells, as the charge holding portions of the micro supercapacitor cells are stretched further apart. **Figure 4c** illustrates the change in capacitance against the degree of linear deformation of the sensor 28 as a linear characteristic, although a skilled person will appreciate that the change in capacitance can be non-linear, based on the design of the sensor 28 and its location on the eyelid respective to the corneal protrusion.

One example of how interface circuitry of the sensor 28 (not illustrated) enables conversion of the location of the deformation 44 of the patch to digital data follows. In the case of a micro supercapacitor cell, an analogue oscillator using the micro supercapacitor cell is a timing capacitor that is connected to a hard limiter. The hard limiter is configured to drive a digital counter. For example, as the underlying flexible substrate 22 comprising the (capacitive) sensor 28 is stretched to be larger in the x direction of Figures 4a and 4b, so the capacitance of the micro supercapacitor cell reduces, thus causing the analogue oscillator to generate hard limited peaks at a higher rate. As the flexible substrate 22 experiences deformation, the (capacitive) sensor 28 above the deformed portion flexible substrate 22 generates a variable count rate at the digital counter. Interface circuitry may sample the count rate at regular intervals to obtain a proxy for the deformation of the flexible substrate 22 at the location of the sensor 28. By tracing the variation of the sampled count rate, a proxy for the REM activity is obtained.

Of course, in more advanced types of detector, the sensor 28 may comprise a 2D sensor matrix capable of identifying the exact location of (for example) the corneal protrusion in 2D coordinates. This this may enable a communicably coupled data analysis algorithm to identify more exact trajectories of the corneal protrusion in time, leading to another way to characterise REM activity.

**Figures 5a** and **5b** illustrate examples of histograms for detecting a REM pattern.

A wide variety of numerical analysis techniques can be used to characterise the presence or absence of REM, based on the type of data received from the sensor 28.

In an embodiment where the sensor 28 is capable of resolving the location of the protrusion or deformation of the eyelid in 2D, an example of features used to characterise the absence or presence of REMS are: horizontal and/or vertical broadband power of the signal characterising the location of the protrusion or deformation of the eyelid. In another example, the horizontal and/or vertical power in different frequency bins may be obtained by performing a fast Fourier transform on a time sampled signal from the sensor 28.

According to an embodiment, the absence or presence of REMS (or the sleep state of the user) is detected according to a sequence of statistical observations of a signal from the sensor 28.

According to an embodiment, the absence or presence of REMS is detected according to the power spectral density of a time sampled signal from the sensor 28.

The article *"*Human Rapid Eye Movement Sleep Shows Local Increases in Low-Frequency Oscillations and Global Decreases in High-Frequency Oscillations Compared to Resting Wakefulness Benjamin Baird", et. al, eNeuro 20 August 2018, 5 (4) ENEURO.0293-18.2018; DOI: 10.1523/ENEURO.0293-18.2018 discusses some variations in the frequency content of eye movement (albeit measured by ECG rather than direct eyelid contact), and observes that sleep cycles associated with REMS show an increased amount of low-frequency content and a decreased amount of high-frequency content.

**Figure 5a** illustrates an example of a frequency histogram of the power spectral density the characterises non-REM eye movement. The power of the high-frequency eye motion components is relatively low, but the power of the high-frequency eye motion components is increased.

**Figure 5b** illustrates an example of a frequency histogram of the power spectral density that characterises REM eye movement. The power of the high-frequency eye motion components has been reduced, but the power of the low-frequency eye motion components is increased.

Accordingly, this gives an opportunity for a REM detection signal processing algorithm to monitor the signals from sensor 28 and classify sleep periods at least as REM, or non REM. Of course, many other REM and non REM classifiers can be applied, dependent on the exact signals obtained from the sensor 28. The patch 20 can directly detect REM eye movement based on monitoring the strain of the eyelid during sleep of the user. This also removes the need for uncomfortable REM monitoring using other devices worn on the head or on the wrist.

The power spectral density histograms for the detection of REM shown in **Figures 5a** and **5b** are exemplary, and many other distributions and statistics can be used to indicate the presence of REMS, or a transition of sleep state.

**Figure 6** schematically illustrates a section view of an example further patch design comprising additional components. Like elements compared to the patch 20 of Figure 1a, 1b and 2 have been described above and will not be described subsequently, save for their interactions with newly introduced elements.

In an embodiment, the patch 20 comprises a photosensor 40. A photosensor converts photons into an electrical current, in order to monitor external lighting conditions. Owing to the small amount of current generated, a skilled person will appreciate that a photosensor is usually considered to comprise associated buffer and amplification circuitry.

According to an embodiment, the patch further comprises a photosensor 40 comprised within the flexible substrate 22, and/or comprised on the second portion 22b of the flexible substrate 22. The photosensor 40 is electronically coupled to the communications interface 30.

The photosensor 40 can, for example, be a portion of a flexible organic photosensor film, deposited on, or close to, the second surface 22b of the flexible substrate 22. The purpose of the photosensor 40 is to output a signal representing the rate at which photons impinge on the photosensor 40. In this way, the photosensor 40 can communicate, via the communications interface 30, an accurate representation of the ambient light level at the closed eyelid of the user.

In an example, the photosensor 40 is encapsulated within the encapsulation layer 27. One type of photosensor 40 comprises providing a portion, or layer, of aromatic polymers onto a substrate of the polymer parylene. The construction of such a thin-film photosensor 40 is detailed in the publication " Ultraflexible Near-Infrared Organic Photodetectors for Conformal Photoplethysmogram Sensors" by Sungjun Park, et. al, in "Advanced Materials", published: 8 July 2018, https://doi.org/10.1002/adma.201802359. Such an ultra-thin device (3um thick) provides durability and fast response times in the near-infrared region, even when stretched or compressed.

The above is one example of photosensor 40, although other types of photosensor 40, including those in small form factor surface mount packages may be provided on, or in, the patch 20. The layer comprising a photosensor may comprise part, or the whole surface of the patch. When the encapsulation layer 27 is transparent or translucent, the photosensor 40 can be embedded within the encapsulation layer 27. In other embodiments, the photosensor 40 is provided on the external surface of the eyelid patch. The photosensor 40 may be of the photo conductor, photovoltaic, or photo gating type as three examples.

In an embodiment, when the photosensor 40 is a portion of a flexible organic photodetector film, such a flexible photosensor 40 can be a polymer, such as a polyindacenodithiophene-pyridylthiadiazole-cyclopentadithiophene (PIPCP) compound, an inorganic material, or a 1D nano structure comprising carbon nanotubes, silicon nano wires, III-V nano wires, metal oxide nano wires, or perovskite nano wires, as some examples.

In an embodiment, the photosensor 40 is active within the visible spectrum (detecting wavelengths of 380 to 750 nm). In an embodiment, the photosensor 40 is active within the infrared spectrum (detecting wavelengths between 10 micrometres and 1mm). In an embodiment, the photosensor 40 is active within the ultraviolet spectrum (detecting wavelengths between one micrometre and one hundred nanometres). The response time of the photosensor 40 can range from 5 ms to 50 ms.

If provided as a layer on a portion of the patch 20, the photosensor layer may range from 1 µm to 20 µm in thickness.

Accordingly, the photosensor 40 is configured to generate a signal characterizing a magnitude and/or a spectral distribution of ambient light incident on the eyelid 10. The photosensor 40 is electronically coupled to the communications interface 30, to enable communication of the signal to the external apparatus 50.

In an embodiment, the patch 20 comprises an electrochromic portion 36.

According to an embodiment, the patch 20 further comprises an electrochromic portion 36. The electrochromic portion 36 is configured to modulate the magnitude and/or spectrum of ambient light able to propagate through the eyelid according to an opacity control signal received via the communications interface 30.

As illustrated in **Figure 6****,** an electrochromic portion 36 is provided as a layer on the side containing the second portion 22b of the flexible substrate 22. In an example, the electrochromic portion 36 is an extension of the encapsulation 27 of the integrated electronics 26. In an example, the electrochromic portion 36 is the same material as the encapsulation 27 of the integrated electronics 26. The purpose of the electrochromic portion 36 is, upon actuation, to reduce, or to prevent ambient light from reaching the eyelid of a user.

For example, the electrochromic portion 36 is capable of reversibly changing its colour or opacity based on the electric potential or current induced in it. When no voltage is applied to the electrochromic portion 36, it is transparent. When a voltage is applied above a threshold voltage value, the electrochromic portion acquires a colour (preferably a dark colour to enhance light attenuation) in the visible spectrum. When the electrochromic portion 36 acquires the colour in the visible spectrum, ambient light is prevented from reaching the user's eyelid and thus waking them up.

In examples, the colour of the electrochromic portion 36, when actuated, is black, dark grey, dark green, dark blue, or dark brown.

In examples, the opacity of the electrochromic portion 36 when actuated is in the range 80% to 100%.

In examples, the colour of the electrochromic portion 36, when not actuated (in other words, when the applied actuation signal is below a threshold voltage), is a lighter colour of the visible spectrum such as yellow or orange, or the electrochromic portion 36 can be transparent.

In examples, when not actuated, the opacity of the electrochromic portion 36 is in the range 0 to 50%.

One example of highly flexible electrochromic devices as discussed in the publication *"*Highly flexible electrochromic devices enabled by electroplated nickel grid electrodes and multifunctional hydrogels" by Zhao, et. al. (Optics Express, Vol. 27, Issue 21, pp. 29547-29557 (2019) https://doi.org/10.1364/OE.27.029547). For example, a triple-layered electrochemical device with high mechanical flexibility was developed using an electroplated nickel grid electrode and multifunctional hydrogel. In that work, the nickel grid electrode with low resistance, high optical transparency, and good mechanical flexibility was useful for efficient electron injection, with a transparent lithium chloride hydrogel functioning as an ion store, ion transport, and counter conductor. A poly(3,4-ethylenedioxythiophene): poly(styrenesulfonate) (PEDOT:PSS) film is utilised as the electrochromic material which distributes the electrons evenly for uniform coloration. The triple-layered electrochromic architecture was concluded not only to simplify the manufacturing procedures, but also improves device performance in terms of optical contrast and mechanical robustness.

A specific example of materials used for electrochromic portion 36 is:
An ion storage, ion transportation, and counter-conducting material comprised of transparent lithium chloride hydrogel, an electrochromic polymer comprising a poly(3,4-ethylenedioxythiophene): poly(styrenesulfonate) (PEDOT:PSS) film, and a transparent electron injection electrode such as an electroplated nickel grid or a tin-doped indium oxide or silver nanowire network.

The transparent electrode is flexible, and may have a transmittance ranging from 80% to 95%. In an example, the transparent electrode is coated on the electrochromic polymer using spin coating. In an example, the thickness of the transparent electrode is between 2 µm to 10 µm. In an example, the thickness of the electrochromic polymer is between 10 µm to 200 µm. In an example, the thickness of the hydrogel layer is between 10 µm to 200 µm.

In an example, the transmittance contrast upon application of the threshold voltage may range from 30% to 60%. The response time of the electrochromic layer (defined as the time to transition between a transparent configuration and an opaque configuration, or vice versa) is in the range 1 second to 10 seconds. The activating voltage for the colour change can range from 0.5 V to 3 V.

In an embodiment, the patch 20 comprises a light emitting portion 34.

According to an embodiment, the patch 20 may further comprise a light emitting portion 34 electronically coupled to the communications interface 30, wherein, in use, the light emitting portion 34 is configured to emit light through the first portion of the flexible substrate and the adhesive layer, upon activation by an illumination control signal via the communications interface 30.

For example, a transparent, elastic, organic light-emitting device (OLED) has been developed (discussed, for example, in Liang, J., Li, L., Niu, X. et al. "Elastomeric polymer light-emitting devices and displays", Nature Photon 7, 817-824 (2013). https://doi.org/10.1038/nphoton.2013.242" or "Full-color active-matrix organic light-emitting diode display on human skin based on a large-area MoS2 backplane" by Minwoo Choi, et. al, in "Science Advances", 8 Jul 2020, Vol 6, Issue 28, DOI: 10.1126/sciadv.abb5898.). The OLED can be repeatedly stretched, folded, and twisted at room temperature while still remaining lit, and retaining its original shape. The proposed material comprises a single layer of an electroluminescent polymer blend sandwiched between a pair of transparent elastic composite electrodes. The electrodes comprise a network of silver nano wires inlaid into a rubbery polymer, which allows the device to be used at room temperatures. All of these layers are fully stretchable, foldable, and twistable. The described material can also be fabricated in relatively simple all-solution-based process. The transparent, elastic composite electrode is stated to have a high visual transparency, good surface electrical conductivity, high stretchability and high surface smoothness.

In an example, the light emitting portion 34 is comprised (mounted) on the second portion 22b of the flexible substrate 22. In an example, the light-emitting portion 34 is encapsulated in an encapsulation layer 27. In this case, the material providing the encapsulation layer may be translucent or transparent to thus provide distribute the emitted light via total internal reflection

For example, the light emitting portion 34 comprises lighting emitting materials such as bioluminescent material, chemiluminescent material, mechanoluminescent material, or electroluminescent material,

In embodiments, the electroluminescent material may be one or a combination of the types of solid state light-emitting diode (LED), organic light-emitting diodes (OLED), polymer light-emitting diodes (PLED), or active-matrix organic light-emitting diodes (AMOLEDs). Light-emitting elements may be located at parts, or the whole of the flexible light-emitting layer. The colour of the light emitted may be monochromatic from any colour of the RGB visible spectrum, or a polychromatic, or white colour.

A specific example of a highly flexible electroluminescent material is a combination of a single layer of flexible electroluminescent polymer blend, and a pair of transparent elastic composite electrodes, like a network of silver nano wires inlaid into a rubbery polymer.

A further specific example of a highly flexible electroluminescent material is a thin-film transistor array, fabricated on a thin transition metal dichalcogenide such as MoS₂, WS₂, MoSe₂, or WSe₂ film transferred to Al₂O₃ or polyethylene terephthalate. Upon the thin-film transistor array, AMOLED pixels are deposited on the thin-film surface.

In an embodiment, the thickness of the light emitting portion 34 ranges between 5 µm to 50 µm.

In an embodiment, the activation voltages of the illuminating pixels at 1 cd m⁻² range from 1 V to 10 V. The luminescence of the pixel may range from 1 to 300 cd m⁻².

In an embodiment, the patch 20 comprises an audio feedback device 37.

In an embodiment, the patch 20 comprises a haptic feedback device 38.

According to an embodiment, the patch 20 or the extension portion further comprise an audio 37 feedback device or a haptic feedback device 38.

For example, the article *"*Transparent and conductive nanomembranes with orthogonal silver nanowire arrays for skin-attachable loudspeakers and microphones" by Saewon Kang, et. al, in "Science Advances", 3 Aug 2018, Vol 4, Issue 8 DOI: 10.1126/sciadv.aas8772 discusses skin adhesive speakers and microphones which are thinner than a temporary tattoo. They are flexible enough to stretch and bend with the skin, without losing capacity to conduct electricity and heat. The skin adhesive speakers discussed in the cited article comprised grids of silver nano wires coated with polymer layers which were stretchy, transparent, and capable of conducting sound signals. After receiving an electric audio signal from music player, the micro-loudspeakers heated up the wire grid to about 33 Celsius, replicating the sound pattern by changing the pressure of the surrounding air. Accordingly, the patch 20 or the extension portion 23 can comprise a skin adhesive speaker and/or microphone. The speaker can be used, for example, to transmit an alarm tone to a user at a wake-up time that has been pre-programmed via a graphical user interface of an external apparatus 50.

For example, the article *"*Miniaturization of mechanical actuators in skin-integrated electronics for haptic interfaces" by Li, D., He, J., Song, Z. et al. in Microsyst Nanoeng 7, 85 (2021). https://doi.org/10.1038/s41378-021-00301-x discusses a class of materials and mechanical designs for the miniaturization of mechanical actuators and strategies for the integration into thin, soft E-skin for haptic interfaces. For example, such haptic actuators may have a diameter of 5 mm, and a thickness of 1.45 mm. The haptic actuators operate in an electromagnetically driven vibrotactile mode with a resonant frequency overlapping the most sensitive frequency of human skin.

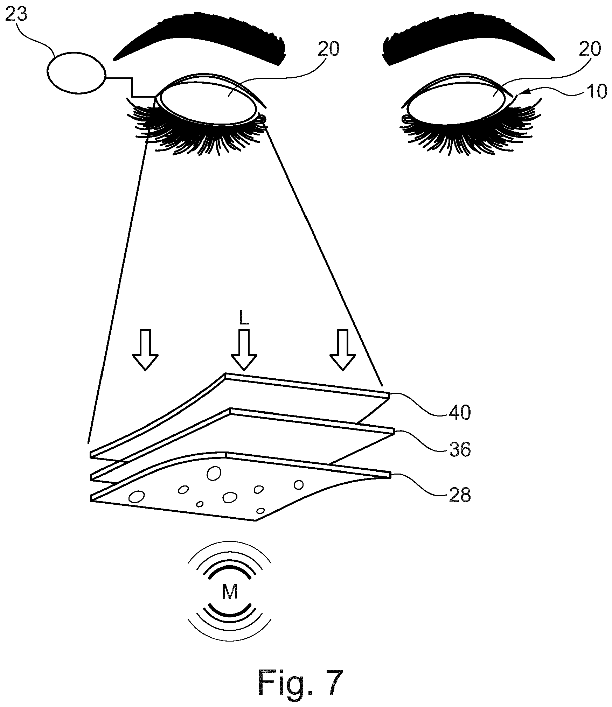

**Figure 7** illustrates a concept according to an example.

In this example, the inset shows the layer construction of a patch 20. A source of motion denoted M impinges on a layer stack comprising at least one sensor 28, an electrochromic portion 36, and a photosensor 40. The photosensor 40 can detect when too much light impinges on the patch 20, and an external apparatus 50 (and/or embedded control electronics integrated into the patch 20) controls the electrochromic portion 36 to thus make both eye patches 20 more opaque. At a wake-up time, and/or when the amount of light has decreased, the external apparatus 50 (and/or embedded control electronics integrated into the patch 20) controls the electrochromic portion 36 to thus make both eye patches 20 more transparent.

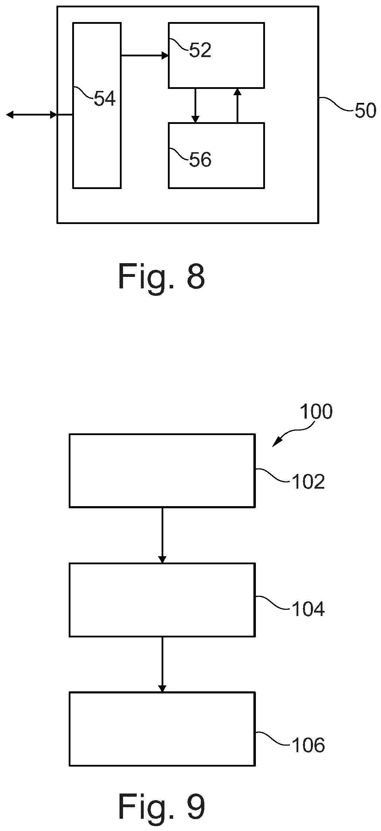

**Figure 8** illustrates an example of an external apparatus 50.

The external apparatus 50 is, in examples, embodied as any computer or processing device capable of performing the computing operations such as REM sleep state analysis based on signals received from the sensor 28, lighting analysis based on signals received from the photosensor 40, sleep protocol generation, and interfacing to one or more patches 20 (in examples, by on-board processing, or by processing performed by a connected web service) within the real-time deadline required by the particular application. For example, the external apparatus 50 can be embodied as a personal computer, a smart tablet, a smartwatch, a smart phone, a cloud server, or web accessible server.

The external apparatus 50 further comprises a processor 52 configured to execute the operating system and application software for communicating and managing the one or more patches 20 of the external apparatus 50, and to coordinate the hardware of the external apparatus 50 such that the user requests (input) to the device are recognised and executed appropriately. The processor 50 comprises, for example, a central processing unit (CPU) and cache memory.

The external apparatus 50 further comprises a communications interface 54. The communications interface 54 is configured to communicate with a local area network, wide area network, by Wi-Fi, Bluetooth, and the like. In particular, the communications interface 54 comprises a means of transferring data to, and receiving data from, one or more patches 20. For example, the integrated electronics 26 of the patches comprises a communications interface 30 that may operate using Near Field Communications (NFC), RFID, Bluetooth (TM), or Zigbee (TM), and thus the communications interface 54 for the external apparatus 50 may also be configured to communicate with the eye patches 20 using one of these protocols. Of course, the listed protocols are indicative, and a skilled person will appreciate that there are many ways of communicating data between the patches 20 and the external apparatus 50.

The external apparatus 50 further comprises memory 56, such as RAM and/or ROM. Also encompassed in the definition of memory is long-term storage such as flash storage or a hard disk drive capable of storing the firmware, and software required for the external apparatus 50 to store, read, and process digital data.

The memory 56 of the external apparatus may comprise machine-readable instructions to execute the steps of the method to be discussed below. Of course, in another embodiment, the integrated electronics 26 of the one or more eye patches 20 may comprise a microprocessor, so that one or more of the steps of the method discussed below can be executed partially on the one or more eye patches 20.

In embodiments, the external apparatus 50 is a smart phone, a personal computer, a laptop computer, a smartwatch, a room environment control system computer, and interactive television, a tablet computer, a personal stereo comprising a computer, an alarm clock comprising a computer, as some examples.

**Figure 9** illustrates an example of a method according to an example of the second aspect.

According to a second aspect, a computer-implemented method 100 for sleep monitoring comprises:
- obtaining 102 one or more signals characterising a shape of an eyelid from a patch 20 according to the first aspect or embodiments as described herein;
- analyzing 104 the one or more signals to determine whether the one or more signals characterize a first or a second phase of sleep of a user; and
- performing 106 at least one output action based on the analysis resulting in a determination that a user of the patch is experiencing either of the first or second phases of sleep.

The description in connection with **Figures 4 and 5** above has already described how a sensor 28 obtains signals or data characterising the shape, or change of shape, of an eyelid from the patch.

Obtaining 102 the one or more signals may comprise, for example, sampling an output of the sensor 28 between first and a second time instants. Dependent on the type of sensor 28 used to measure the shape, or change of shape, of an eyelid, a variety of signals may be measured, such as a resistance of the sensor 28, a capacitance of the sensor 28, or an inductance of the sensor 28, and the like. In an example of a sensor 28 comprising a micro supercapacitor array, the measured signal is a magnitude of one or more stored voltages, for example.

According to an embodiment, obtaining 102 the one or more signals comprises sampling an output of the sensor 28 at least at first and second time instants.

In an example, when the sensor 28 comprises a 2D matrix sensing element, the obtained signal may be a multidimensional (non-scalar) signal. Accordingly, the process of obtaining 102 the signal characterising a shape of an eyelid captures signals characterising the deformation 44 of the eyelid illustrated in **Figure 4** between first and a second time instant.

According to an embodiment, the integrated electronics 26 comprised in the patch 20 perform pre-processing on the one or more signals obtained in step 102. The pre-processing may comprise, for example, analogue filtering, signal routing, analogue digital conversion, digital filtering, digital feature extraction as some examples. In this case, the integrated electronics 26 can comprise analogue and/or digital circuitry to perform the pre-processing.

According to an embodiment, the integrated electronics 26 comprised in the 20 provide the (optionally pre-processed) one or more signals to a communications interface, so that the signal characterising a shape of an eyelid can be communicated to an external apparatus 50.

According to an embodiment, the integrated electronics 26 comprises analogue circuitry and the communications interface 30 comprises an analogue amplitude modulated, or frequency modulated transmitter. An analogue output from the sensor 28 representing a shape, and/or a change in shape, of an eyelid is used to directly amplitude and/or frequency modulate a carrier wave generated by the communication interface 30. The modulated carrier wave is received by a communications interface 54 of an external apparatus 50. In this embodiment, therefore, eyelid motion signals detected by the patch are used to directly modulate an amplitude and/or frequency carrier, and analogue to digital conversion of the eyelid motion signal is performed at the external apparatus 50. Such an example can operate with a lower power consumption at the patch 20, with energy-intensive digital signal processing performed by the external apparatus 50.

Therefore, the step of analysing 104 the one or more signals output by sensor 28 can be performed either by the integrated electronics 26 of the patch 20, by a combination of the patch 20 and an external apparatus 50, or exclusively by the external apparatus 50.

According to an embodiment, the step 104 of analysing the one or more signals output by sensor 28 comprises peak and/or trough detection, or zero crossing detection, of the one or more signals output by sensor 28. The analysis can, for example, detect an increased rate of zero crossing as a proxy for entering a REMS state.

According to an embodiment, the step 104 of analysing the one or more signals output by sensor 28 comprises spectral monitoring (such as using frequency histogram) of the one or more signals. The analysis, for example, can use the detection of one or more spectral signatures as a proxy for entering a REMS state.

According to an embodiment, the step 104 of analysing the one or more signals output by sensor 28 comprises monitoring a motion trajectory of the deformation of the eyelid (when the sensor 28 comprises a 2D matrix distributed over a portion of surface of the eyelid). The analysis, for example, can use the detection of one or more trajectory shapes, or their power (amplitude), as a proxy for entering a REMS state.

Of course, the analysis performed in the analysis step is dependent on the type of sensor 28 applied on the patch, its shape, and on differences of the monitor user.

According to an embodiment, the method comprises a step of calibrating the sensor 28 of the patch 20 and/or analysis software in the external apparatus 50. For example, a user may wear a patch 20 and simultaneously perform eye movements (such as up, down, left, right) according to instructions given by the external apparatus 50. The external apparatus 50 may receive such calibration eye movement signals from the sensor 28, and calibrate the patch based on the calibration eye movement signals and a comparison to expected signals at the external apparatus, so that more accurate REMS detection can be performed.

Accordingly, the integrated electronics 26 of one or more eye patches 20, and/or an external apparatus 50, can be configured to receive the signals or data characterising the shape of an eyelid, and/or a change in shape of the eyelid. A signal processing operation forming a REM detection algorithm on the signal received from the one or more eye patches 20, to classify the current sleep state as a REM state, or another sleep state. Of course, monitoring the eye movement may enable detection of other sleep states that are not REM sleep states.

According to an embodiment, the first phase of sleep is non-REM sleep, and the second phase of sleep is REM sleep.

An example protocol using the eye patch monitoring system is stated as follows. Initially, a user decides to go to sleep. The user obtains two eye patches and attaches the eye patches to his or her eyelids. The user configures application software to be executed by the external apparatus 50 via a graphical user interface, such as a smart phone application. The user then begins to sleep. The eye patches 20 (potentially in combination with the external apparatus 50) monitor for the presence of REMS. If REMS is detected, the external apparatus 50 updated a sleep state model. In an example, if REMS is detected, the performance one or more output actions associated with a wake up protocol is changed.

As an example, if REMS is detected during a programmed wake-up time, the external apparatus 50 delays one or more wake-up actions (such as illuminating the light-emitting portion 34, causing the electrochromic portion to become transparent, signalling to a radio or a blind or curtain opening mechanism and the like). This provides a sleep extension, and prevents the user from waking up during a REMS portion of the sleep cycle. A user can programme a terminal time of the sleep extension period, so that the system wakes up a user during a REMS portion of the sleep cycle after the terminal time of the sleep extension, to prevent the user from oversleeping.

At the time that the external apparatus 50 determines that the user should wake up, the external apparatus 50 performs one or more wake-up actions (such as illuminating the light-emitting portion 34 of the eye patches, causing the electrochromic portion of the eye patches to become transparent, signalling to a radio or a blind or curtain opening mechanism, generating audio or haptic feedback, and the like).

According to an embodiment, the output action comprises delaying, or accelerating, a wake-up time of a user of the patch.

According to an embodiment, the output action comprises transmitting an illumination control signal to a light emitting portion 34 comprised on the patch 20. For example, the light emitting portion is activated, such that the user experiences an enhanced level of light at their eyelids. In an example, the enhanced level of light is applied gradually.

According to an embodiment, the output action comprises transmitting an opacity control signal to an electrochromic portion 36 of the patch 20. For example, the opacity of the patch is decreased. In an example, the opacity of the patch is decreased gradually.

According to an embodiment, the output action comprises transmitting an audio or haptic feedback signal to an audio feedback device 37 or haptic feedback device 38 of the patch.

According to an embodiment, the output action comprises transmitting a control signal to an external apparatus 50.

According to an embodiment, the output action comprises transmitting the control signal to a stand-alone audio device in order to provide audio feedback to wake-up the user. For example, the audio device may be a loudspeaker, a personal stereo, an alarm clock, a personal computer, and the like.

According to an embodiment, the output action comprises transmitting the control signal to a stand-alone lighting device and an LED light bulb comprising a digital controller and communication modem, or an intelligent room lighting system.

According to an embodiment, the output action comprises transmitting the control signal to a stand-alone haptic device, so that the user can experience haptic or vibration feedback. For example, the control signal may be transmitted to a smartwatch worn by the user.

According to an embodiment, the method comprises receiving, via a user interface 52, an operating protocol specifying an output action to perform using the patch 20, based on the presence of the first or second phase of sleep of the user, a time of day, and/or ambient lighting conditions.

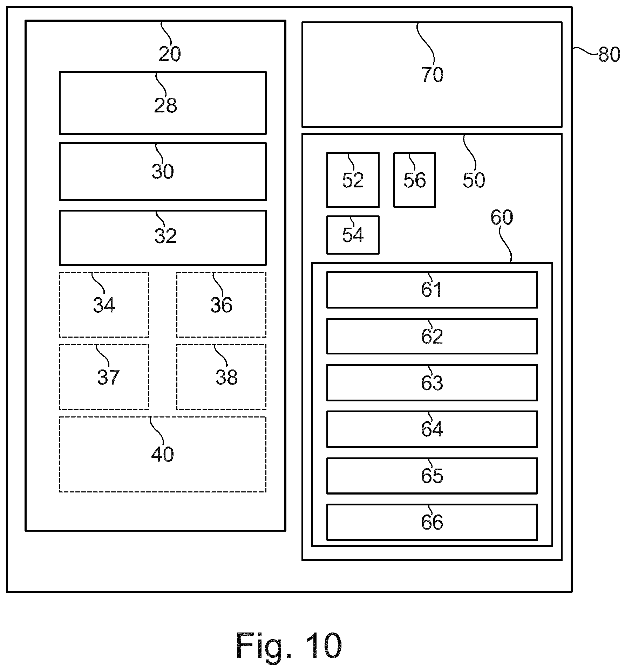

**Figure 10** illustrates an example of a system according to an example of the third aspect. Where appropriate, like reference numerals are used to denote a previously described elements.

According to a third aspect, a system 80 comprises at least one patch 20 according to the first aspect, or embodiments discussed herein. The system further comprises an external apparatus 50 comprising a processor 52, a communications interface 54, and a memory 56.

The communications interface 54 of the apparatus is configured to obtain one or more signals characterising a shape of an eyelid 10 from the at least one patch 20.

The processor 52 is configured to analyze the one or more signals to determine whether the one or more signals characterize a first or a second phase of sleep of a user, and the processor 52 is configured to transmit a command to perform at least one output action based on the analysis resulting in a determination that a user of the patch is experiencing either of the first or second phases of sleep.

In an embodiment, communications interface 30 of the patch 20 and the communications interface 54 of the external apparatus 50 are configured to communicate using, for example, Near Field Communications (NFC), RFID, Bluetooth (TM), or Zigbee (TM). In an embodiment, communications interface 30 of the patch 20 and the communications interface 54 of the external apparatus 50 are configured to communicate using analogue wireless communication such as AM and/or FM.

In use, the external apparatus 50 may be initialised, and configured, using the processor 52, to load a software environment 60 from memory 56. For example, the software environment 60 is an operating system such as Android (TM), or Apple iOS (TM). In turn, the software environment 60 is configured to host application software 61 for coordinating the one or more patches 20.

The application software 61 is configured to handle communication with a user (such as displaying historical sleeping patterns, or receiving a wake-up protocol) via a graphical user interface 70, and to establish and to maintain communications with one or more patches 20 and the external apparatus 50. The application software 61 is configured to generate one or more control signals and to communicate the one or more control signals to one or more of the patch 20, and other external items such as alarm clocks, room lighting control systems, and the like via the communications interface 54 of the external apparatus 50. In other words, the application software 61 is application software providing a user interface 70 to a user and encompassing all software modules necessary for the operation and communication of the external apparatus 50 with one or more patches 20. The user interface 70 is capable of receiving one, or more, operating protocols of the patch 20 from a user.

The software environment 60 is configured to host a REM detection engine 62. The REM detection engine 62 is configured to receive one or more signals characterising a shape, or a change of shape, of an eyelid from a patch 20 according to the first aspect or embodiments. Dependent on the type of sensor 28 used in the patch 20, a variety of signal processing algorithms can be applied to the one or more signals characterising shape, or a change of shape, of the eyelid. As discussed previously, the signal processing algorithms can vary from the relatively simple (such as a monitoring the rate of peaks appearing in a one-dimensional signal from the sensor 28), to a spectral analysis of a one-dimensional signal, or more complicated trajectory tracking of a two-dimensional signal. Trained classifiers can be applied to the signal received from the sensor 28 of the patch 20, in order to distinguish between different sleep states. Accordingly, the output of the REM detection engine 62 provides a binary or probabilistic signal defining which of several sleep states a user of the patch 20 is considered to be in. In a basic case, the REM detection engine 62 distinguishes between a REM sleep state and a non REM sleep state, although by analysis of the input signals, could also classify other states of deep sleep.

In an exemplary embodiment, the software environment 60 is configured to host a light monitoring engine 63. The light monitoring engine 63 receives a signal representing the external light intensity, as monitored by a photosensor 40 of the patch 20, and/or another sensor in the environment in which the user of the patch 20 sleeping. The light monitoring engine 63 is configured to perform pre-processing and analysis of the signal representing external light intensity.

When an electrochromic portion 36 is present on the patch 20, the light monitoring engine 63 may generate an electrochromic portion control signal based on the determination of ambient light derived by the light monitoring engine 63. According to an embodiment, the light monitoring engine 63 is configured to communicate with a sleep state monitoring engine 66, and/or a feedback engine 65, so that one or more output actions can be taken based on the monitored light level. For example, if the light monitoring engine 63 detects a high level of ambient light present at the photosensor 40 of the patch 20 at a time late at night, this indicates that a user might have fallen asleep with a light in the room switched on. In this case, the light monitoring engine 63 could instruct the application software 61 to search in a home automation database or lighting control system for a light that has been accidentally left on, and if found, to turn off that light automatically.

In an exemplary embodiment, the software environment 60 is configured to operate a patch 20 set up engine 64. The patch set up engine 64, for example, communicates with one or more patches 20 that have been recently registered and a communication network to obtain identification information. The setup engine 64 can update the mobile software 61 based on the type of patches 20 that have been registered. For example, a different instance of the REM detection engine 62 needs to be operated based on the type of sensor 28 of a particular type of patch 20.

In an exemplary embodiment, the software environment 60 is configured to operate a feedback engine 65. The feedback engine 65 obtains, for example, an output of the sleep state monitoring engine 66. The feedback engine 65 is configured to generate a wake-up feedback signal, via the patch 20 by communicating a control signal to another component of the patch 20 (such as the light emitting portion 34). In another embodiment, the feedback engine 65 is configured to communicate a control signal to a stand-alone device, such as an alarm clock, a room lighting system, alarm application of a smartphone, curtain control system, amongst many possibilities.

The software environment 60 is configured to operate a sleep state monitoring engine 66. The sleep state monitoring engine 66 obtains, for example, an output of the REM detection engine 62. If the REM detection engine 62 defines that the user is not in a REM sleep state, then the sleep state monitoring engine 66 logs the time for a user is not in a REM sleep state. If the REM detection engine 62 defines that the user is in a REM sleep state, the sleep state monitoring engine 66 logs the time that the user is in a REM sleep state. Over the length of a night, the sleep state monitoring engine builds a characteristic of a user's sleep state that can be applied to a wake-up algorithm. For example, a user may desire not to be woken up during a REM sleep phase, on the condition that by delaying the wake-up time until the end of the REM sleep phase, the user will not be woken up beyond a limit time (final alarm clock time).

According to an embodiment, the system comprises an output device configured to receive the output signal. The output device is one of: a smartphone, a tablet computer, a personal computer, a personal stereo system, a room lighting system, an alarm clock, an automatic curtain control system, a haptic output system, and/or an automatic blind control system.

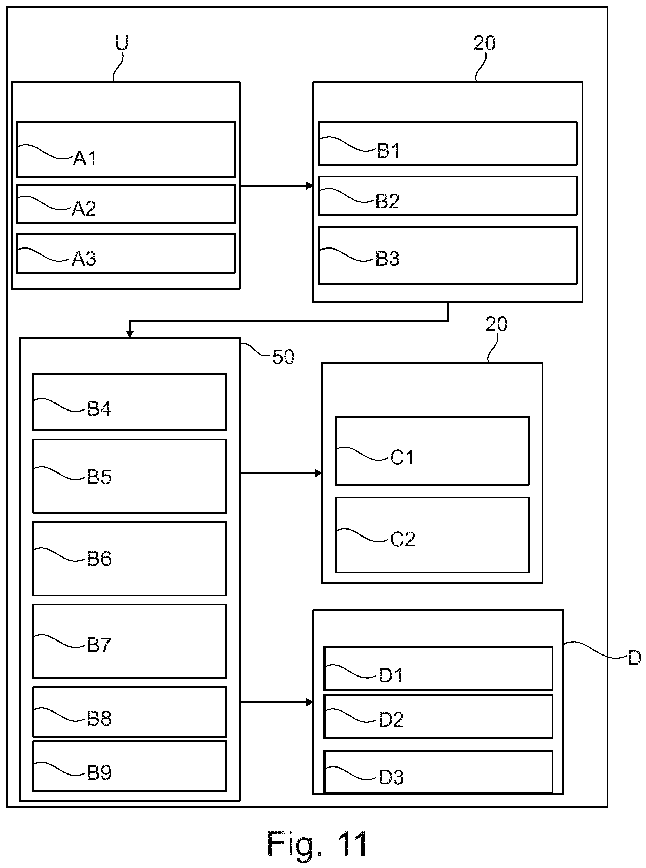

**Figure 11** illustrates an example of the system according to the third aspect, in use.

A user (U) sets the wake-up protocol through the mobile device application (A1). The user then fits two eyelid patches 20, one to each eye (A2). It is noted for the basic functionality of REM sleep phase monitoring, only one eyepatch 20 needs to be fitted. Then, the user gets into their bed and falls asleep (A3).

The eyelid patches 20 are configured to monitor eye movements (B1), and in some examples to monitor ambient lighting conditions (B2). The eyelid patches 20 communicate information (B3) concerning the eye movement, such as one or more signals characterising a shape of the flexible substrate, and in some examples ambient lighting conditions, to an external apparatus 50.

The external apparatus 50 is configured to receive (B4) at least the information concerning the eye movement (one or more signals characterising a shape of the flexible substrate). The external apparatus 50 evaluates REM eye movements (B5) using the REM detection engine 62. In some examples, the external apparatus 50 evaluates ambient lighting conditions (B6) using a light monitoring engine 63. If the feedback engine 65 determines that an action should be performed based on the REM eye movements, the feedback engine 65 causes the communications interface 54 of the external apparatus 52 to communicate (B7) one or more commands to perform one or more output actions. An example, the command may be sent (B8) to the two eyelid patches 20, or to a stand-alone device (B9). As a result of receiving the command, the eyelid patches 20 may, for example, change opacity (C1) to shield the user from ambient light, or initiate (C2) a light emitting portion 34 of the patches 20 as part of a wake-up protocol. Alternatively or in addition, supporting devices, or the patches 20, may receive a command to initiate an audio signal (D1), a haptic signal (D2), and/or a visual signal (D3). According to an embodiment, the REM detection engine 62 continues to monitor signals from the eyepatches, and to adapt the signals based on the response of the user. For example, if a wake-up sound appears not to be rousing a user, the volume of the wake-up sound can be further increased until the user begins to wake.

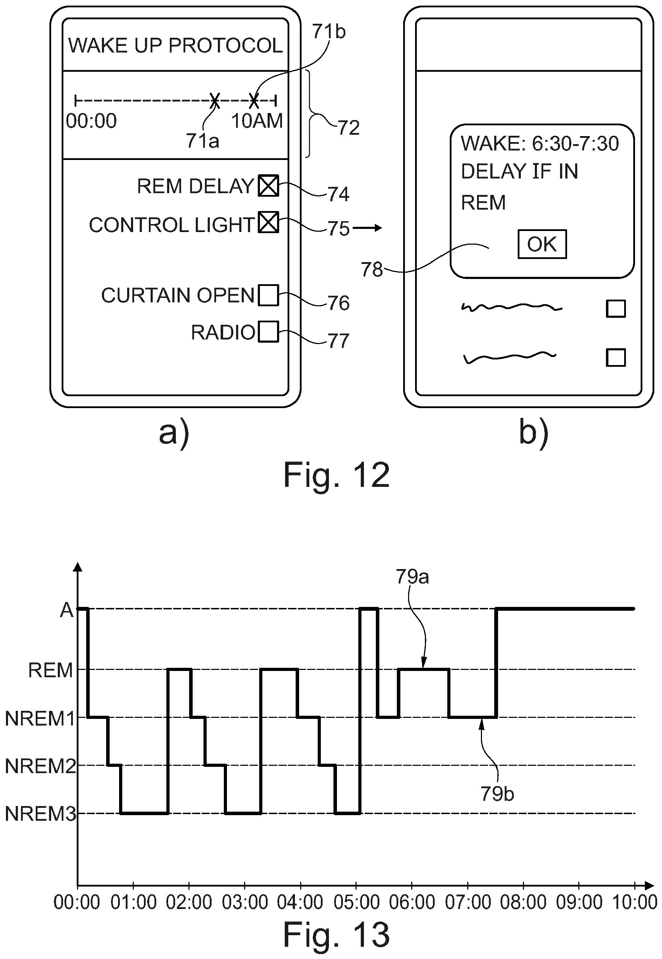

**Figures 12a****-b** illustrates an example of a graphical user interface.

The graphical user interface is illustrated as being displayed on a screen having typical smartphone proportions, although it will be appreciated that the graphical user interface can be adapted to other screen formats, such as that of a smart phone, PC screen, smart alarm clock screen, and the like.

The graphical user interface comprises a display element enabling a user to set a wake-up protocol. In the illustrated case, a first slider 71a defines a start time of a wake-up period, and a second slider 71b defines an end time of a wake-up period. The user has further specified that the "REM delay" feature and the "Control light" feature should be activated, and that the "curtain open" and "radio" features should not be activated.

The selection of the "REM delay" feature means that if the REM detection engine 62 determines that a user is experiencing a REM sleep cycle within the time defined between first slider 71a and second slider 71b a wake-up protocol involving initiating a light (for example, the light emitting portion 34 of the patches 20) will be delayed until the REM detection engine 62 determines that a user has finished experiencing a REM sleep cycle based on the signals detected by the sensor 28 of the patch 20. This offers the possibility of allowing the user a more natural wake-up phase. However, if the REM sleep cycle continues beyond the time defined by the second slider 71b, the "REM delay" feature is overridden, and the light emitting portion 34 of the patches 20 are activated anyway. This provides a failsafe that ensures a sleeping user does not wake-up too late. Once the wake-up protocol defined in the graphical user interface has been selected, the user is shown a summary of the wake-up protocol, which they confirm using a confirmation dialog box 78.

A skilled person will appreciate that many different wake-up protocols can be defined based on the selection of output modalities available, and that the illustration of the wake up protocol in the GUI of **Figure 12****,** and the foregoing description, is only one example.

**Figure 13** illustrates an example of a hypnogram.

The sleep state monitoring engine 66 of the application software 61 can generate a hypnogram as one example of monitoring the sleep state of a user. The REM detection engine 62 detects when a user is experiencing REMS, and can also classify other states of deep sleep (NREM1, NREM2, NREM3) analysis of the signals characterising a shape of the flexible substrate obtained from the sensors 28 of the patches 20. Following the wake-up protocol discussed in **Figure 12****,** for example, the sleep state monitoring engine 66 prevents the wake-up protocol from being initiated at time point 79a, because this point the user is still experiencing REMS. However, by time point 79b, the sleep state monitoring engine 66 is able to securely identify that the recent REMS has ended, and therefore the wake-up protocol is then initiated.

According to a fourth aspect, a computer program element comprises machine readable instructions which, when executed by a processor, causes the processor to perform the computer implemented method of the second aspect.

According to a fifth aspect, a computer readable medium comprises the computer program element according to the fourth aspect.

References throughout the preceding specification to "one embodiment", "an embodiment", "one example" or "an example", "one aspect" or "an aspect" means that a particular feature, structure, or characteristic described in connection with the embodiment or example is included in at least one embodiment of the present disclosure. Thus, appearances of the phrases "in one embodiment", "in an embodiment", "one example" or "an example", "one aspect" or "an aspect" in various places throughout this specification are not necessarily all referring to the same embodiment or example.

Furthermore, the particular features, structures, or characteristics can be combined in any suitable combinations and / or sub-combinations in one or more embodiments or examples.

### REFERENCE NUMERALS

| | | | |
|---|---|---|---|
| U | User | 50 | External apparatus |
| L | Light | 52 | Processor |
| M | Motion due to eye movement | 54 | Communications interface |
| 10 | eyelid | 56 | Memory |
| 20 | Patch | 60 | Software environment |
| 21 | Backing | 61 | Application software |
| 22 (a,b) | Flexible substrate | 62 | REM detection engine |
| 23 | Extension portion | 63 | Light monitoring engine |
| 24 | Adhesive layer | 64 | Patch setup engine |
| 26 | Integrated electronics | 65 | Feedback engine |
| 27 | Integrated electronics encapsulation | 66 | Sleep state monitoring engine |
| 28 | Eyelid shape sensor | 70 | User Interface |
| 30 | Communications interface | 72 | Wake up protocol slider |
| 32 | Power source | 74 | Delay selector |
| 33 | Power and/or communication wire | 75 | Lighting selector |
| 34 | Light emitting portion | 76 | Curtain selector |
| 36 | Electrochromic portion | 77 | Radio selector |
| 37 | Audio feedback device | 78 | Confirmation dialog |
| 38 | Haptic feedback device | 80 | System |
| 40 | Photosensor | 100 | Method |
| 42 | Strip of patches | 102 | Obtaining |
| 44 | Deformation of patch | 104 | Analyzing |
| | | 106 | Performing |

## Claims

1. A patch (20) for eyelid monitoring, comprising:
- a flexible substrate (22) configured for attachment to at least a portion of an external surface (10) of an eyelid (12);
- an adhesive layer (24) comprised on a first portion (22a) of the flexible substrate (22), wherein the adhesive layer (24) is configured, in use, to adhere to the external surface (10) of the eyelid (12), to enable a semi-permanent attachment of the flexible substrate (22) to the external surface (10) of the eyelid (12); and
- integrated electronics (26) comprised at least partially within the flexible substrate (22), and/or comprised on a second portion (22b) of the flexible substrate (22) on an opposite side of the flexible substrate to the first portion (22a);
wherein the integrated electronics (26) comprises:
a sensor (28) configured to generate one or more signals characterising a shape of the flexible substrate (22);
a communications interface (30) configured to communicate the one or more signals to an external apparatus (50); and
a power source (32) configured to provide electrical energy to the sensor (28) and the communications interface (30).

2. The patch (20) according to claim 1,
wherein the sensor (28) is configured to detect motion of the external surface of the eyelid (12) caused by a motion of an eyeball.

3. The patch (20) according to claims 1 or 2,
wherein the sensor (28) is a strain sensor configured to generate the one or more signals characterising strain induced in the flexible substrate (22) owing to deformation of the eyelid (10) caused by movement of an underlying eyeball (12), in use.

4. The patch (20) according to one of the preceding claims, further comprising:
- a photosensor (40) comprised within the flexible substrate (22), and/or comprised on the second portion (22b) of the flexible substrate (22), and wherein the photosensor (40) is electronically coupled to the communications interface (30);
wherein the photosensor (40) is configured to generate a signal characterizing a magnitude and/or a spectral distribution of ambient light incident on the eyelid (10), and wherein the photosensor (40) is electronically coupled to the communications interface (30), to enable communication of the signal to the external apparatus (50).

5. The patch (20) according to one of the preceding claims, further comprising:
- a light emitting portion (34) electronically coupled to the communications interface (30);
wherein, in use, the light emitting portion (34) is configured to emit light through the first portion of the flexible substrate and the adhesive layer, upon activation by an illumination control signal via the communications interface (30).

6. The patch (20) according to one of the preceding claims, further comprising:
- an electrochromic portion (36);
wherein the electrochromic portion (36) is configured to modulate the magnitude and/or spectrum of ambient light able to propagate through the eyelid according to an opacity control signal received via the communications interface (30).

7. The patch (20) according to one of the preceding claims,
wherein the flexible substrate (22) further comprises an extension portion (23) that that does not contact the eyelid, in use, and at least one of the power source (32), and/or the communications interface (50) are comprised on the extension portion (23).

8. The patch (20) according to claim 7, further comprising:
- an audio (37) or haptic feedback device (38) comprised on the extension portion (23).

9. A computer-implemented method (100) for sleep monitoring, comprising:
- obtaining (102) one or more signals characterising a shape of an eyelid from a patch (20) according to one of claims 1 to 8;
- analyzing (104) the one or more signals to determine whether the one or more signals characterize a first or a second phase of sleep of a user; and
- performing (106) at least one output action based on the analysis resulting in a determination that a user of the patch is experiencing one of the first or second phases of sleep.

10. The computer-implemented method (100) according to claim 9,
wherein obtaining 102 the one or more signals comprises sampling an output of the sensor 28 at least at first and a second time instants.

11. The computer-implemented method (100) according to one of claims 9 or 10, wherein the at least one output action is selected from one, or more, of:
- delaying, or accelerating, a wake-up time of the user;
- transmitting an illumination control signal to a light emitting portion (34) comprised on the patch (20);
- transmitting an opacity control signal to an electrochromic portion (36) of the patch (20);
- transmitting an audio or haptic feedback signal to an audio feedback device (37) or haptic feedback device (38) of the patch; and/or
- transmitting a control signal to an external apparatus (50).

12. The computer-implemented method (100) according to one of claims 9 to 11, further comprising:
- receiving, via a user interface (70), an operating protocol specifying an output action to perform using the patch (20), based on the presence of the first or second phase of sleep of the user, a time of day, and/or ambient lighting conditions.

13. A system (80) comprising:
- at least one patch (20) according to one of claims 1 to 8; and
- an external apparatus (50) comprising:
- a processor (52);
- a communications interface (54), and
- a memory (56);
wherein the communications interface (54) of the apparatus is configured to obtain one or more signals characterising a shape of an eyelid (10) from the at least one patch (20), wherein the processor (52) is configured to analyze the one or more signals to determine whether the one or more signals characterize a first or a second phase of sleep of a user; and wherein the processor (52) is configured to transmit a command to perform at least one output action based on the analysis resulting in a determination that a user of the patch is experiencing either of the first or second phases of sleep.

14. The system (80) according to claim 13, further comprising:
- an output device configured to receive the output signal, wherein the output device is one of: a smartphone, a tablet computer, a personal computer, a personal stereo system, a room lighting system, an alarm clock, an automatic curtain control system, a haptic output system, and/or an automatic blind control system.

15. A computer program element comprising machine readable instructions which, when executed by a processor, causes the processor to perform the computer implemented method of one of claims 9 to 12.
